Europäisches Patentamt

European Patent Office

Office européen des brevets

(11) Numéro de publication: **0 323 327**
**A1**

## DEMANDE DE BREVET EUROPEEN

(21) Numéro de dépôt: **88403292.1**

(22) Date de dépôt: **22.12.88**

(51) Int. Cl.⁴: **A 61 B 6/00**

(30) Priorité: **29.12.87 FR 8718300**

(43) Date de publication de la demande:
**05.07.89 Bulletin 89/27**

(84) Etats contractants désignés: **DE ES GB IT NL**

(71) Demandeur: **GENERAL ELECTRIC CGR S.A.**
**13, Square Max-Hymans**
**F-75015 Paris (FR)**

(72) Inventeur: **Trotel, Jacques**
**Cabinet Ballot-Schmidt 84 Avenue Kléber**
**F-75116 Paris (FR)**

(74) Mandataire: **Ballot, Paul Denis Jacques et al**
**Cabinet Ballot-Schmit 84, avenue Kléber**
**F-75116 Paris (FR)**

(54) Statif de radiologie isocentrique.

(57) L'invention concerne un statif de radiologie, permettant des mouvements isocentriques et destiné notamment au radiodagnostic.

Le statif (1) comporte un socle (2) supportant un étrier (24) ayant un premier et un second bras (28,29) portant respectivement une source et un récepteur (25,26) de rayons X.

Selon une caractéristique de l'invention, l'étrier (24) comporte un pied (27) par lequel il est supporté par le socle (2), par l'intermédiaire de moyens (4,11,89,90) pour déplacer le pied (27) en un mouvement circulaire ayant pour centre un point isocentre (IC).

FIG.1

EP 0 323 327 A1

## Description

## STATIF DE RADIOLOGIE ISOCENTRIQUE

L'invention concerne un statif de radiologie, destiné notamment au radiodiagnostic et permettant une exploration ou examen isocentrique d'un patient avec des incidences multiples.

L'examen radiologique d'un patient s'effectue à l'aide d'une chaine image qui, généralement, est constituée essentiellement d'une source de rayons X, d'un colimateur, d'une grille anti-diffusante et d'un récepteur d'image qui sont portés par le statif et liés rigidement entre eux; la source de rayons x et le collimateur sont d'un même coté par rapport au patient à examiner, et la grille anti-diffusante et le récepteur d'image sont du côté opposé. Une droite passant par le centre de la source de rayonnement x et le centre du récepteur d'image représente l'axe du rayonnement x ou axe de la chaine image qui, dans le cas d'un examen ou exploration isocentrique passe toujours en un même point d'une zone à analyser, quelle que soit l'orientation de cet axe, ce point constituant l'isocentre ; le mouvement que permet de faire varier l'orientation de l'axe de la chaine image en gardant une position fixe par rapport à l'isocentre étant appelé mouvement isocentrique.

Les statifs qui permettent de réaliser un mouvement isocentrique comportent, généralement, un arceau ouvert dont une extrémité porte une source de rayons x, et l'autre extrémité porte un récepteur d'image. L'axe de la chaine image constitué entre le récepteur d'image et la source de rayons x passe par l'isocentre, lequel isocentre constitue le centre de l'arceau ou est sur un même axe que le centre de l'arceau de sorte qu'un mouvement isocentrique est réalisé de faisant tourner l'arceau sur lui-même dans son plan, autour de son centre, en faisant coulisser l'arceau dans un fourreau en forme d'arc de cercle par exemple.

Un agencement semblable est utilisé aussi bien dans le cas de systèmes radiologiques lourds et importants où les mouvements de l'arceau sont motorisés, que dans le cas de petits systèmes radiologiques, tels que mobiles chirurgicaux ou mammographes par exemple, ou la chaine image n'est pas d'une masse trop élevée, de sorte qu'il n'est pas nécessaire que les mouvements de l'arceau soient motorisés ; ces mouvement pouvant être obtenus par simple action manuelle de l'opérateur.

On note en outre que ces différents statifs de radiologie permettent en outre un autre mouvement isocentrique qui consiste en une rotation du plan de l'arceau autour d'un second axe de rotation perpendiculaire au premier, et passant également par l'isocentre.

L'un des défauts d'un tel agencement réside dans le fait que, quand l'axe de la chaine image est parallèle à ce second axe, on n'obtient aucune modification de son angle d'incidence à l'aide du second mouvement isocentrique, c'est-à-dire en réalisant une rotation du plan de l'arceau autour du second axe de rotation.

Un autre inconvénient de cet agencement, particulièrement pour les systèmes plus petits non motorisés tels que les mobiles chirurgicaux par exemple, est que les mouvements de l'arceau par simple action manuelle de l'opérateur ne peuvent être obtenus que par un rééquilibrage du système, c'est-à-dire en plaçant des contrepoids à l'extérieur du système, ce qui les rend particulièrement encombrants. On peut noter, pour souligner ce problème, que dans certains de ces petits systèmes dont l'arceau est déplacé sous une action manuelle, la source de rayonnements x et le récepteur d'image sont portés chacun à une extrémité opposée de l'arceau, à des positions très extérieures de ce dernier, c'est-à dire telles que l'axe de la chaine image n'est plus sur un diamètre de l'arceau. Ceci apporte une solution au problème de l'équilibrage, mais présent l'inconvénient d'exiger un mouvement supplémentaire de translation, à chaque nouvelle incidence obtenue par la rotation de l'arceau, pour que l'axe de la chaine image passe par l'isocentre.

Il est à observer aussi qu'un autre inconvénient de ces statifs isocentriques, réside en ce qu'il est nécessaire de leur ajouter des moyens importants et encombrants pour substituer l'un à l'autre la source et le récepteur de rayonnements x, par un mouvement isocentrique, tout en conservant une même orientation de l'axe de la chaine image.

La présente invention concerne un statif de radiologie, permettant des mouvements isocentriques et qui ne présente pas les inconvénients ci-dessus cités. Selon l'invention, un statif de radiologie comportant, un socle, un étrier ayant deux bras, le premier et le second bras portant respectivement une source et un récepteur de rayons x, la source et le récepteur de rayons x définissant entre eux un axe de chaine image passant par un isocentre, et caractérisé en ce que l'étrier comporte un pied, et en ce que le pied est porté par le socle par l'intermédiaire de moyens pour déplacer le pied dans un mouvement circulaire ayant l'isocentre pour centre. L'invention sera mieux comprise grace à la description qui suit faite à titre d'exemple non limitatif et aux sept figures annexées, parmi lesquelles:

- les figures 1 à 3 montrent de manière schématique une première version d'un statif de radiologie conforme à l'invention ;

- la figure 4 montre schématiquement un dispositif d'orientation d'un étrier montré aux figures 1 à 3 ;

- la figure 5 montre schématiquement un dispositif permettant de réaliser un déplacement relatif en translation entre deux bras destinés à porter l'étrier dans la première version de l'invention ;

- la figure 6 montre schématiquement une seconde version d'un statif de radiologie conforme à l'invention ;

- la figure 7 montre schématiquement un second dispositif d'orientation de l'étrier ;

- la figure 8 montre schématiquement une troisième version d'un statif selon l'invention ;
- la figure 9 montre schématiquement un dispositif pour conserver une orientation constante à un bras montré à la figure 8.

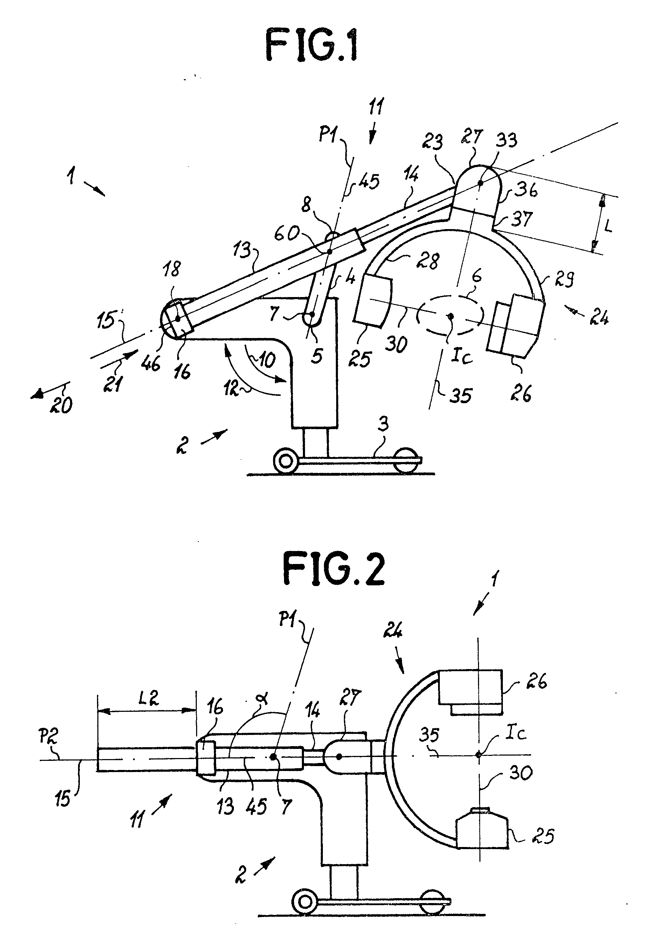

La figure 1 représente un statif de radiologie 1 conforme à une première version de l'invention qui met en oeuvre un principe d'homothétie.

Le statif de radiologie 1 comporte un socle 2. Dans l'exemple non limitatif décrit, le socle 2 est monté sur un chariot 3. Une bielle 4 est montée sur le socle 2 par une extrémité 5 de la bielle 4 de sorte que la bielle 4 soit libre en rotation par rapport au socle 2, autour d'un axe de rotation 7 (perpendiculaire au plan de la figure) selon lequel l'extrémité 5 est fixée au socle 2. La seconde extrémité 8 de la bielle 4 peut ainsi décrire, autour de l'axe de rotation 7, un mouvement circulaire dans l'un au l'autre des sens représentés par une première et une seconde flèches 10, 12.

La seconde extrémité 8 de la bielle 4 est fixée à une colonne télescopique 11. Dans l'exemple non limitatif décrit la colonne télescopique 11 est formée par deux bras 13,14 que peuvent être déplacés l'un par rapport à l'autre en translation, le long d'un axe longitudinal 15 de la colonne télescopique 11. Le socle 2 comporte un fourreau 16 dans lequel est engagé et peut coulisser le premier bras 13 de la colonne télescopique 11. La seconde extrémité 8 de la bielle 4 est fixée au premier bras 13, libre en rotation par rapport à ce dernier, de manière à permettre à la fois : le mouvement circulaire de la bielle 4 autour de l'axe de rotation 7 ; et un mouvement circulaire du premier bras 13 autour d'un second axe de rotation 18 (perpendiculaire au plan de la figure), passant sensiblement par le centre du fourreau 16 ; ce mouvement circulaire du premier bras 13 autour du second axe de rotation 18 étant combiné avec un déplacement dans le fourreau 16 du premier bras 13, déplacement qui s'effectue dans l'un ou l'autre des sens montré par une troisième et une quatrième flèches 20,21 (par rapport à l'axe longitudinal 15). Les mouvements accomplis par le premier bras 13 sont également accomplis par le second bras 14 qui, en outre, est mobile par rapport au premier bras 13 le long de l'axe longitudinal 15, et dans un même sens 20, 21 que le premier bras 13.

Une extrémité 23 du second bras 14, opposée au premier bras 13, porte un étrier 24 qui lui-même porte une source et un récepteur 25, 26 de rayonnements X ; la source et le récepteur 25, 26 étant situé de part et d'autre d'un patient 6 à examiner. Dans l'exemple non limitatif décrit, l'étrier 24 a la forme d'un diapason ayant un pied 27 et deux branches 28, 29 dont la première 28 porte la source 25, et la seconde 29 porte le récepteur 26. La source et le récepteur de rayonnements x 25,26 définissent entre eux, de manière classique, un axe 30 de chaine image. L'axe de chaine image 30 passe par un point IC qui constitue l'isocentre.

Le pied 27 est fixé à l'extrémité 23 du second bras 14 de manière à être libre en rotation par rapport à ce dernier ou, plus précisément par rapport à un troisième axe de rotation 33, perpendiculaire au plan contenant les deux branches 28,29 et au plan de la figure.

Dans l'exemple non limitatif décrit, le pied 27 a une longueur L entre l'extrémité 23 du second bras 14 et les deux branches 28,29, et cette longueur L est disposée sensiblement selon un axe 35 constituant un quatrième axe de rotation 35 passant par l'isocentre 31. En effet, dans l'exemple non limitatif décrit, le pied 27 de l'étrier 24 comporte une première et une seconde partie 36, 37 liées entre elles par des moyens de roulement classique (non représentés), de sorte que la seconde partie 37 attachée aux deux branches 28,29 puisse tourner autour de quatrième axe de rotation 33, par rapport à la première partie 36 qui est attachée à l'extrémité 23 du second bras 14.

Dans cette configuration, le mouvement circulaire de la seconde extrémité 8 de la bielle 4, autour du premier axe de rotation 7, est transformé en un mouvement circulaire du pied 27 autour d'un centre constitué par l'isocentre IC.

Le statif radiologique 1 comporte d'autre part, un dispositif (non représenté sur la figure 1) pour orienter l'étrier 24 en fonction de l'orientation de la bielle 4, c'est-à-dire de sorte que, d'une part, le point de fixation du pied 27 sur le second bras 14 (point de fixation qui est représenté par le troisième axe de rotation 3), et d'autre part l'isocentre IC se déduisent respectivement de l'extrémité 8 de la bielle 4 ou plus précisément d'un point de fixation 60 de cette dernière sur le premier bras 13, et du premier axe de rotation 7. Dans l'exemple de cette première version de l'invention, ceci est obtenu par une homothétie dont le centre est le second axe de rotation 18, le rapport d'homothétie étant par exemple de 2.

La figure 1 représente le statif 1 dans une première position P1 de la bielle 4, la bielle 4 étant alors levée, la position P1 étant confondue avec un second axe longitudinal 45 de la bielle 4. Dans cette première position P1 de la bielle 4, la colonne télescopique 11 est à un position extrême de sa course, c'est-à-dire qu'une extrémité 46 du premier bras 13 est sensiblement au niveau du fourreau 16. D'autre part, l'étrier 24 est orienté pour que l'axe de chaine image 30 et la bielle 4 conservent de mêmes orientations relatives, de sorte que l'axe 30 de chaine image passe toujours par l'isocentre IC ; la bielle 4 étant perpendiculaire à l'axe de la chaine image 30, dans l'exemple non limitatif décrit.

La figure 2 montre le statif 1 conforme à la première version de l'invention, dans une seconde position P2 sensiblement horizontale de la bielle 4, après un mouvement circulaire de cette dernière accompli autour du premier axe de rotation 7 dans le sens de la première flèche 10, selon un angle α un peu supérieur à 90°. Dans l'exemple non limitatif décrit, la bielle 4 n'est pas visible sur le figure 2 du fait qu'elle est montée dans un plan plus profond que le plan de la colonne télescopique 11, par rapport au plan de la figure, et se trouve masquée par la colonne télescopique 11 qui est également horizontale, et les deux axes longitudinaux 15,45 aparaissent confondus. La colonne télescopique 11 est alors engagée au maximum dans le fourreau 16, c'est-à-dire que l'extrémité 46 du premier bras 13 dépasse du fourreau 16 d'une seconde longueur L2 maximum. L'axe 30 de la chaine image passe toujours par l'isocentre IC, mais son orientation qui est maintenant verticale a été modifiée d'une même quantité que l'orientation de la bielle 4.

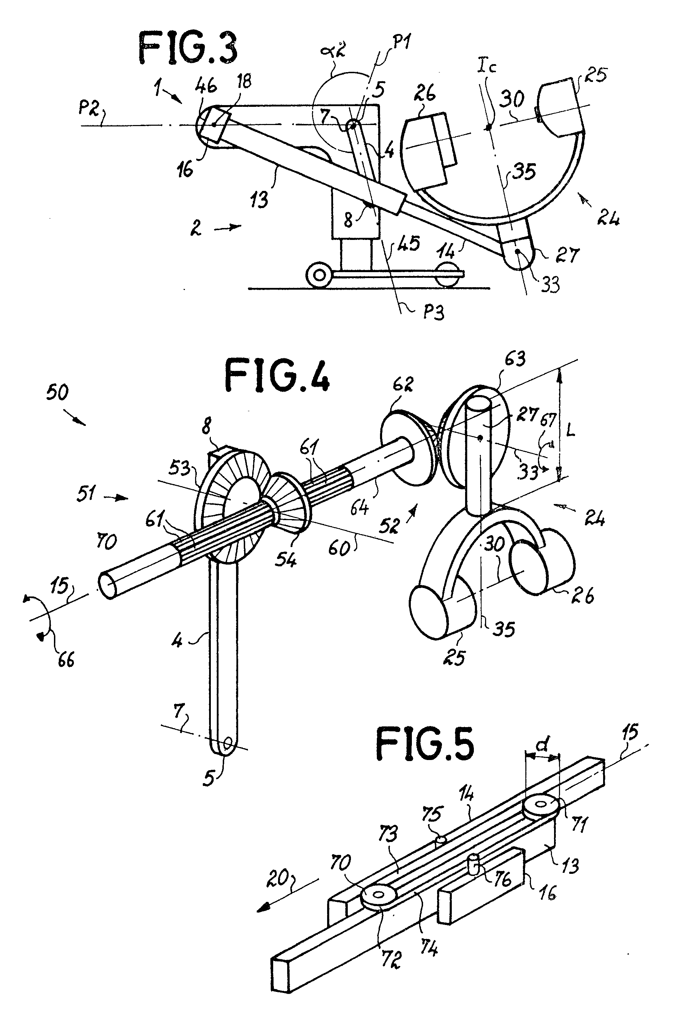

La figure 3 montre le statif 1 de la première version pour une troisième position P3 de la bielle 4, obtenue en poursuivant le mouvement circulaire de cette dernière dans le sens de la flèche 10 ; la colonne télescopique 11 étant à nouveau à une position extrême de sa course, c'est-à-dire que l'extrémité 46 du premier bras 13 est revenu au niveau du fourreau 16. entre la première position P1 correspondant à la situation de la figure 1, et la troisième position P3 correspondant à la situation de la figure 3, l'orientation de la bielle 4 a été modifiée d'un angle $\alpha 2$ plus grand que 180°, et l'orientation de l'axe 30 de chaine image a été modifié dans le même sens et d'une même quantité. Ceci illustre un des avantages de l'invention qui est de pouvoir remplacer l'un par l'autre la source et le récepteur de rayonnements x 25,26, une rotation de 180° de la bielle 4 étant suffisant à cet effet. On peut noter d'autre part que pour toutes les positions possibles entre les position P1 et P3, l'orientation de l'axe 30 de chaîne images peut être modifiée par une rotation des deux branches 28,29 autour du quatrième axe de rotation 35.

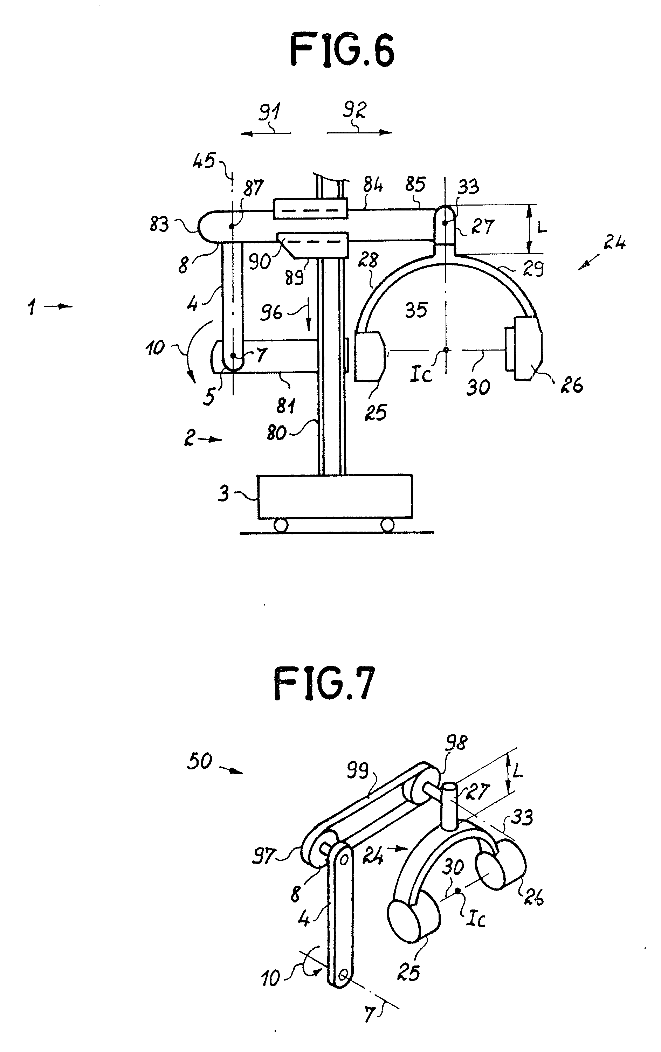

La figure 4, montre de manière schématique un dispositif 50 pour réaliser l'orientation de l'étrier 24. Dans l'exemple non limitatif décrit, le dispositif d'orientation 50 comporte un premier et un second engrenage 51,52 montés respectivement à la seconde extrémité 8 de la bielle 4 et à l'extrémité du pied 27, comme il est représenté sur la figure 4. Le premier engrenage 51 comporte un premier et un second pignon conique 53, 54. Le premier pignon 53 est monté à l'extrémité 8 de la bielle 4 et le second pignon 54 est monté sur un axe support 64 lié au second bras 14 (non représenté sur la figure 4). Le second pignon 54 peut coulisser sur l'axe support 64 le long de l'axe longitudinal 15 ; l'axe support 64 comportant à cet effet des canelures 61 parallèles à l'axe longitudinal 15. Cette disposition permet le coulissement du second bras 14 par rapport au premier bras 13 tout en maintenant le second pignon 54 en contact avec le premier pignon 53. Le second engrenage 52 est formé d'un troisième pignon cônique 62 monté à l'extrémité de l'axe support 64, et ce troisième pignon 62 est en prise avec un quatrième pignon 63 monté sur le pied 27. Comme il apparaît sur la figure 4, les engrenages 51,52 sont montés de telle sorte que dans les mouvements conjugués ci-dessus décrits du premier et du second bras 13, 14 et de la bielle 4, d'une part le second pignon 54 coulisse le long des canelures 61 et tourne sur le premier pignon 53 autour d'un centre qui correspond à l'axe de fixation 60 (selon lequel la seconde extrémité 8 de la bielle 4 est fixée sur la colonne télescopique 11) ; ce dernier mouvement du second pignon 54 entraînant la rotation de l'axe support 64 sur lui-même comme représenté par la flèche 66. D'autre part, cette dernière rotation de l'axe support 64 entraîne la rotation du troisième pignon 62 qui, lui-même provoque un mouvement de rotation du quatrième pignon 63 et par conséquent du pied 27 autour du troisième axe de rotation 33, comme représenté par la flèche 67 ; de sorte que la longueur L du pied 27 et le cinquième axe de rotation 35 sont toujours sensiblement parallèles à la bielle 4.

La figure 5 montre schématiquement, à titre d'exemple non limitatif, comment sont liés l'un à l'autre, le premier et le second bras 13,14 pour obtenir leur mouvement relatif et constituer la colonne télescopique 11. Le premier bras 13 porte une première et une seconde poulie 70,71 qui sont disposées le long de l'axe longitudinal 15 et qui sont montées libres en rotation par rapport au premier arbre 13. Les deux poulies 70,71 sont reliées par une courroie 72 qui est formée autour de ces deux poulies ; de sorte qu'entre les deux poulies 70,71 la courroie 71 a une première et une seconde partie rectilignes 73, 74 , parallèles à l'axe longitudinal 15, et qui sont séparées l'une de l'autre par le diamètre d d'une poulie. La première partie rectiligne 73 est solidaire d'un pion 75 fixé à la seconde barre 14, et la seconde partie rectiligne 74 est solidaire d'un second pion 76 lui-même solidaire d'une partie fixe qui représente le fourreau 16. Dans ces conditions, un mouvement du premier bras 13 par rapport au fourreau 16, dans le sens de la troisième flèche 20 par exemple, provoque un déplacement du second bras 14 par rapport au premier bras 13, et dans un même sens et avec une même amplitude que pour ce dernier, c'est à dire avec une amplitude double par rapport au fourreau 16.

La figure 6 montre à titre d'exemple non limitatif, une seconde version du statif de radiologie 1 conforme à l'invention.

Le statif 1 comporte une colonne 80. Dans l'exemple non limitatif de la description, la colonne 80 est verticale, et elle porte un bras support 81 portant lui-même la bielle 4 déjà citée. La bielle 4 est fixée par son extrémité 5 au bras support 81, de manière à être libre en rotation par rapport à ce dernier autour du premier axe de rotation 7. La seconde extrémité 8 de la bielle 4 est fixée à une extrémité 83 d'un quatrième bras 84, dont la seconde extrémité 85 porte le pied 27 de l'étrier 24.

Comme précédemment expliqué, l'étrier 24 a une première et une seconde branche 28, 29 qui supportent respectivement la source et le récepteur de rayonnement x 25,26, et l'axe 30 de la chaine image passe par le point isocentre IC. La seconde extrémité 8 de la bielle 4 et la première extrémité 83 du quatrième bras 84, sont assemblées l'une à l'autre de manière à pouvoir être articulées autour d'un cinquième axe de rotation 87 perpendiculaire au plan de la figure. Le pied 27 de l'étrier 24 est fixé à la seconde extrémité 85 du quatrième bras 84, de manière à pouvoir tourner autour du troisième axe de rotation 33 et, comme dans l'exemple précédent, le statif de radiologie est agencé pour que le pied 27 et plus précisément le troisième axe de rotation 33 soit capable d'un mouvement circulaire selon un cercle dont le centre est l'isocentre IC.

A cette fin, dans cette seconde version de l'invention, le statif de radiologie comporte, d'une part un second chariot 89, mobile le long de la colonne 80, et comporte d'autre part un second fourreau 90 monté sur le chariot 89 et dans lequel est engagé le quatrième bras 84. Le quatrième bras 84 est ainsi porté par le second fourreau 90 dans lequel il peut coulisser transversalement par rapport à la colonne 80, dans l'un ou l'autre des sens montrés par les flèches 91,92.

On remarque que d'une part, le bras support et le quatrième bras 81,84 sont parallèles et que d'autre part, sont parallèles également, la bielle 4 et la longueur L du pied 27 ; de sorte que ces éléments parallèles deux à deux représentent un parallélogramme et, qu'en maintenant fixe le bras support 81 et en déformant le parallèlogramme on obtient la rotation du pied 27 autour de l'isocentre IC.

En effect, en supposant que le quatrième bras 84 coulisse dans le second fourreau 90, dans le sens de la sixième flèche 91 par exemple, ce mouvement provoque un mouvement de rotation de la bielle 4 autour du premier axe de rotation 7 dans le sens de la première flèche 10, et en même temps un mouvement du chariot 89 le long de la colonne 80 dans le sens d'une huitième flèche 96.

Il est ainsi possible de modifier l'orientation de l'axe 30 de la chaine image d'une manière aussi importante que dans l'exemple précédent et il est à remarquer que cette version de l'invention se prête particulièrement bien à un équilibrage des différents mouvements.

Bien entendu la rotation du pied 27 autour de l'isocentre IC doit être accompagnée d'une orientation de l'étrier 24, de sorte que la longueur grand L du pied 27 soit toujours sensiblement parallèle à la bielle 4 ; ceci étant obtenu à l'aide du dispositif d'orientation 50 (non visible sur la figure 6) qui, dans cette version de l'invention est particulièrement simple.

La figure 7 montre de manière schématique et à titre d'exemple non limitatif, une réalisation du dispositif 50 servant à orienter l'étrier 24 dans le cadre de la seconde version de l'invention montrée à la figure 6.

La seconde extrémité 8 de la bielle 4 porte une première roue 97 et le peid 27 porte une seconde roue 98. Les deux roues sont liées entre elles par une courroie 99 crantée par exemple, qui, entre les deux roues 97, 98 est parallèle au quatrième bras 84 (montré sur la figure 6). Une rotation de la bielle 4 autour du premier axe de rotation 7, dans le sens par exemple montré par la première flèche 10, entraîne un déplacement de la courroie 99 qui provoque une rotation du pied 27 autour du troisième axe de rotation 33 ; les mouvement de rotation de la bielle 4 et du pied 27 ayant un même sens et une même amplitude, de sort que la longueur L du pied 27 reste parallèle à la bielle 4.

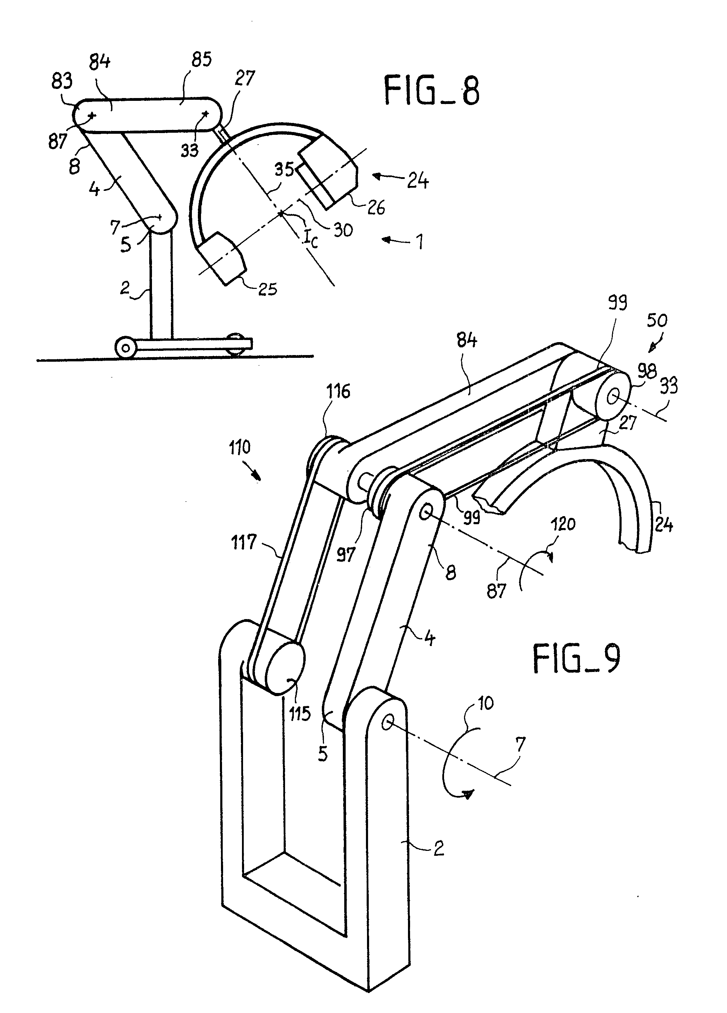

La figure 8 montre à titre d'exemple non limitatif, une troisième version du statif de radiologie 1 selon l'invention.

La bielle 4 est portée par le socle 2 auquel elle est fixée par son extrémité 5, de manière à être libre en rotation autour de premier axe de rotation 7. La seconde extrémité 8 de la bielle 4 peut ainsi décrire un mouvement circulaire autour du premier axe de rotation 7, comme dans les exemples précédents; ce mouvement circulaire est transmis au pied 27 de l'étrier 24 d'une même manière que dans la seconde

version décrite en référence à la figure 6, c'est à dire à l'aide du quatrième bras 84.

A cette effet, la seconde extrémité 8 de la bielle 4 est fixée à une extrémité 83 du quatrième bras 84, dont la seconde extrémité 85 porte le pied 27 de l'étrier 24.

A la condition d'orienter l'étrier 24, et à la condition de maintenir constante l'orientation du quatrième bras 84, par rapport au socle 2 par exemple (orientation horizontale dans l'exemple non limitatif décrit), un mouvement circulaire de la première bielle 4 autour du premier axe de rotation 7 dans le sens de la première flèche 10 par exemple, engendre un mouvement circulaire de l'étrier 24 selon un cercle ayant pour centre l'isocentre IC. Il est ainsi possible de modifier l'orientation de l'axe 30 de la chaine image d'une manière aussi importante que dans les exemples précédents.

La figure 9 est une vue en perspective qui montre, à titre d'exemple non limitatif, à la fois une réalisation du dispositif 50 servant à orienter l'étrier 24 dans le cadre de la troisième version de l'invention montré à la figure 8, et une réalisation d'un dispositif 110 servant à maintenir constante l'orientation du quatrième bras 84.

Le premier dispositif d'orientation 50 est d'un même type que celui décrit en référence à la figure 7. La première bielle 4 est fixée par sa première extrémité 5 au socle 2 de manière à être libre en rotation autour du premier axe de rotation 7. La seconde extrémité 8 de la bielle 4 porte la première roue 97 et le pied 27 de l'étrier 24 (partiellement représenté) porte la seconde roue 98. Les deux roues sont liées entre elles par la courroie 99. Une rotation de la bielle 4 autour du premier axe de rotation 7, dans le sens par exemple montré par la première flèche 10, entraine un déplacement de la courroie 99 qui provoque une rotation du pied 27 autour du troisième axe 33 et entraine l'orientation appropriée de l'étrier 24.

Le second dispositif d'orientation 110 déstiné à conserver une orientation constante au second bras 84, est réalisé à l'aide d'une troisième et d'une quatrième poulie 115,116 reliées entre elles par une seconde courroie 117. La troisième et quatrième poulies 115,116 sont montées respectivement sur le socle 2 et à la première extrémité 83 du quatrième bras 84, de sorte que la seconde courroie 117 est tendue entre ces deux poulies 115,116 parallèlement à la première bielle 4.

Une rotation de la première bielle 4 autour du premier axe de rotation 7, dans le sens de la première flèche 10, entraine un déplacement de la seconde courroie 117, qui elle-même entraine un mouvement de rotation de quatrième bras 84, par rapport à la première bielle 4, autour du cinquième axe de rotation 87 selon lequel sont assemblés la bielle et le quatrième bras 84. Ce mouvement de rotation du quatrième bras 84 est effectué dans un sens représenté par une flèche 120, ce sens de rotation étant approprié à conserver son orientation au quatrième bras 4 malgrès le mouvement de la bielle 4.

**Revendications**

1. Statif de radiologie comportant un socle (2,80), un étrier (24) ayant deux bras (28, 29) le premier et le second bras (28, 29) portant respectivement une source (25) et un récepteur (26) de rayonnements X, la source et le récepteur (25, 26) définissent entre eux un axe (30) de chaîne image passant par un isocentre (IC), caractérisé en ce que l'étrier (24) comporte un pied (27) qui est porté par le socle (2,80) par l'intermédiaire de moyens (4, 11, 89, 90) pour déplacer le pied (27) selon un mouvement circulaire ayant pour centre l'isocentre (IC) et en ce que lesdits moyens pour déplacer le pied (27) comportent une bielle (4) ayant une extrémité (5) fixée au socle (2,80) et une seconde extrémité (8) libre en rotation par rapport au socle (2,80) autour d'un axe de rotation (7) passant par la première extrémité (5), le mouvement circulaire du pied (27) étant engendré par la rotation de la bielle (4) autour de l'axe de rotation (7).

2. Statif de radiologie selon la revendication 1, caractérisé en ce qu'il comporte en outre un dispositif (50) pour maintenir une orientation relative constante entre le pied (27) et la bielle (4).

3. Statif de radiologie selon la revendication 2, caractérisé en ce que le pied (27) et la bielle (4) sont parallèles.

4. Statif de radiologie selon l'une des revendications précédentes, caractérisé en ce que le pied (27) est porté par une colonne télescopique (15) coopérant avec la bielle (4) et tournant autour d'un centre d'homothétie (18).

5. Statif de radiologie selon l'une des revendications 1 à 3, caractérisé en ce que le pied (27) est mécaniquement relié à la seconde extrémité (8) de la bielle (4) par l'intermédiaire d'un bras (84) mobile ayant une orientation constante par rapport au socle (2,80).

6. Statif de radiologie selon la revendication 5, caractérisé en ce que le bras (84) est mobile en translation selon eux axes orthogonaux (80, 84).

7. Statif de radiologie selon la revendication 5, caractérisé en ce que le socle (2) est formé par une colonne (80), la colonne (80) portant un chariot (89) mobile le long de la colonne (80), le chariot (89) portant ledit bras (84), et en ce que ledit bras (84) est mobile par rapport au chariot (89) transversalement à la colonne (80).

# FIG.1

# FIG.2

# FIG.3

# FIG.4

# FIG.5

# FIG.6

# FIG.7

FIG_8

FIG_9

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | EP-A-0 160 749 (SIEMENS AG) <br> * Résumé; page 3, ligne 16 - page 4, ligne 3; page 4, ligne 23 - page 5, ligne 11; page 5, lignes 21-26; page 5, ligne 31 - page 6, ligne 8; figures 1-5 * | 1 | A 61 B 6/00 |
| A | | 4-7 | |
| | --- | | |
| Y | CH-A- 426 094 (PHILIPS) <br> * En entier * | 1 | |
| | ----- | | |

| DOMAINES TECHNIQUES RECHERCHES (Int. Cl.4) |
|---|
| A 61 B <br> F 16 M |

Le présent rapport a été établi pour toutes les revendications

| Lieu de la recherche | Date d'achèvement de la recherche | Examinateur |
|---|---|---|
| LA HAYE | 13-03-1989 | FERRIGNO, A. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
................................................................
& : membre de la même famille, document correspondant

EPO FORM 1503 03.82 (P0402)